# EUROPÄISCHE PATENTSCHRIFT

(11) **EP 1 926 810 B1**
(45) Veröffentlichungstag und Bekanntmachung des Hinweises auf die Patenterteilung: **02.04.2014**
(21) Anmeldenummer: 06805681.1
(22) Anmeldetag: 08.09.2006
(51) Int. Cl.: C12M 1/107

(54) **BIOREAKTOR MIT RÜCKHALTESYSTEM**
BIOREACTOR COMPRISING A RETAINING SYSTEM
BIOREACTEUR EQUIPE D'UN SYSTEME DE RETENUE

(30) Priorität: 08.09.2005 DE 202005014176 U
(43) Veröffentlichungstag der Anmeldung: 04.06.2008
(73) Patentinhaber: Bekon Energy Technologies GmbH & CO. KG, 85774 Unterföhring (DE)
(72) Erfinder: LUTZ, Peter, 81927 München (DE)
(74) Vertreter: Winter, Brandl, Fürniss, Hübner, Röss, Kaiser, Polte - Partnerschaft
(86) Internationale Anmeldenummer: PCT/EP2006/008794
(87) Internationale Veröffentlichungsnummer: WO 2007/028642

(56) Entgegenhaltungen:
- EP-A2- 0 023 176
- EP-B1- 1 301 583
- DE-A1- 10 302 658
- DE-U1- 20 203 533
- DE-U1- 20 319 847

## Beschreibung

Die Erfindung betrifft einen Bioreaktor zur Methanisierung von Biomasse.

Ein Bioreaktor der hier vorgestellten Art ist aus der EP 1 301 583 B1 bekannt, auf deren Offenbarung zur Vermeidung unnötiger Wiederholung vollinhaltlich Bezug genommen und deren Offenbarungsgehalt ausdrücklich in die vorliegende Anmeldung einbezogen wird.

Aus der EP 0023176 A2 ist ein Bioreaktor zur Methanisierung von Biomasse bekannt. Dieser Bioreaktor umfasst einen kubischen oder rohrförmigen Faulbehälter, der durch eine Klappe mittels einer elastischen Dichtung (gasdicht verschließbar ist. Hinter der Klappe ist eine Gitterbox vorgesehen, die mit der zu vergärenden Biomasse befüllt ist. Damit stellt die Gitterbox eine Druckentlastung für die gasdicht ausgeführte Klappe dar und Biomasse stützt sich vollständig an der Gitterbox ab.

Die DE 202 03 533 U1 offenbart einen Bioreaktor zur Biogaserzeugung in einem gasdicht verschließbaren Faulbehälter. Der Boden des Faulbehälters ist dabei einstückig in Richtung der Klappe abschüssig ausgebildet und mit einem Radlader befahrbar. Hinter der Klappe des Bioreaktors befindet sich eine Sickersaft-Drainageeinrichtung.

Ausgehend von dem Bioreaktor nach der DE 202 03 533 U1 ist es Aufgabe der vorliegenden Erfindung einen mit einem Radlader befahrbaren Bioreaktor dahingehend zu verbessern, dass er große Beladung an Biomasse aufnehmen kann ohne die Dichtungseigenschaften der Dichtungsklappe negativ zu beeinflussen.

Die Lösung dieser Aufgabe erfolgt durch einen Bioreaktor nach Anspruchs 1.

Durch die gasdicht verschließbare Klappe, die ausreichend groß ausgeführt ist, kann auf einfache Weise Biomasse in den Behälter eingefüllt und die Biorestmasse kann nach der Methanisierung leicht wieder entnommen werden. Die Biomasse drückt bei geschlossenem Behälter im wesentlichen oder wenigstens zu einem bestimmten Anteil auf das Rückhaltesystem. Hierdurch wird die Klappe entlastet und kann gleichermaßen leicht und präzise abdichtend ausgeführt sein. Zwischen Klappe und Rückhaltesystem ist im Boden oder im Boden und den Wänden des Bioreaktors eine Sickersaft- bzw. Perkolat-Drainageeinrichtung angeordnet. Hierdurch kann Sickersaftansammlung im Bereich zwischen Klappe und Rückhaltesystem vermieden werden bzw. vor dem Öffnen der Klappe kann dort befindliches Perkolat abgepumpt werden.

Vorteilhafterweise ist das Rückhaltesystem flüssigkeitsdurchlässig. Die Biomasse drückt unter ihrem Eigengewicht von innen gegen das Rückhaltesystem, so dass Sickersaft aus- und durch das Rückhaltesystem gepresst wird, hinter dem es in der zwischen Rückhaltesystem und Klappe im Boden angeordneten Drainageeinrichtung gesammelt und abgeführt wird.

In einer bevorzugten Ausführung reicht das Rückhaltesystem vom Boden des Faulbehälters bis zu einer bestimmten Teilhöhe oder auch alternativ über die gesamte vertikale Höhe des Faulbehälters. Das Rückhaltesystem besteht aus einer oder mehreren im wesentlichen plattenförmigen Elementen, die in entsprechende vertikale Führungsschienen eingesetzt werden.

Das Rückhaltesystem kann, wie gesagt, im wesentlichen aus einem oder mehreren vertikalen plattenförmigen Elementen bestehen. Diese können sich in einer ersten Ausführung über die gesamte horizontale Breite des Faulbehälters erstrecken. In einer alternativen Ausführung kann der Faulbehälter an seiner durch die Klappe verschließbaren Stirnseite auch eine durch das Rückhaltesystem verschließbare Öffnung aufweisen, die sich nicht über die gesamte Breite des Faulbehälters erstreckt.

Gemäß einer vorteilhaften Ausgestaltung der Erfindung ist die den Faulbehälter gasdicht abschließende Klappe mit einem aufblasbaren Dichtungsschlauch versehen. In geschlossenem Zustand wird der Dichtungsschlauch aufgeblassen und dichtet die Klappe gegenüber der Behälterwandung auf einfache Weise gasdicht ab.

Gemäß einer weiteren vorteilhaften Ausgestaltung der Erfindung ist die Klappe hydraulisch betätigbar, da diese bei entsprechenden Dimensionen kaum mehr von Hand betätigbar ist.

Gemäß einer vorteilhaften Ausgestaltung der Erfindung ist der Faulbehälter kubisch oder quaderförmig ausgeführt, wobei die Klappe eine wandung des Kubus oder Quaders bildet. Hierdurch ergibt sich zum einen eine einfache Konstruktion und zum anderen eine ausreichend große Öffnung zum Beladen und Befüllen des Faulbehälters. Zusätzlich vereinfacht sich dadurch die Herstellung des Faulbehälters.

Gemäß einer vorteilhaften Ausgestaltung der Erfindung ist die Decke des Faulbehälter mittels Hubzylindern anhebbar und wieder gasdicht verschließbar. Hierdurch wird eine schnelle Belüftung des Faulbehälter gewährleistet.

Gemäß einer vorteilhaften Ausgestaltung der Erfindung ist der Faulbehälter zylindrisch ausgebildet und die Klappe besitzt die Form eines scheibenförmigen Deckels. Diese Form ist insbesondere für Rundstrohballen als Biomasse geeignet.

Gemäß einer vorteilhaften Ausführungsform der Erfindung ist die Heizeinrichtung nach Art einer Fußbodenheizung in die Bodenplatte Faulbehälters integriert, da warme Gase nach oben steigen, wird hierdurch eine gleichmäßige Durchwärmung der Biomasse in dem Faulbehälter erreicht. Zusätzlich oder alternativ lässt sich die Heizeinrichtung auch in die übrige Behälterwandung integrieren.

Gemäß einer weiteren vorteilhaften Ausgestaltung der Erfindung ist der Faulbehälter nach Art einer Fertiggarage aus Stahlbeton aufgebaut. Die offene Seite der "Fertiggarage" wird durch die Klappe gasdicht verschlossen. Hierdurch ergibt sich eine sehr kostengünstige Konstruktion.

Weitere Einzelheiten, Merkmale und Vorteile der Erfindung ergeben sich aus den Unteransprüchen und der nachfolgenden Beschreibung bevorzugter Ausführungsformen. Hierzu zeigt:
Es zeigt:
   Fig. 1 schematisch eine perspektivische Darstellung des erfindungsgemäßen Bioreaktors ohne eingesetztes Rückhaltesystem;
   Fig. 2 eine Schnittdarstellung des Bioreaktors nach Fig. 1;
   Fig. 3 eine schematische Darstellung der Bodenplatte des Bioreaktors aus Fig. 1 bzw. 2;
   Fig. 4a eine Aufsicht auf die Klappe des Faulbehälters;
   Fig. 4b eine Schnittdarstellung der Klappe aus Fig. 4a entlang der Ebene A-A;
   Fig. 4c ein Detail der Darstellung in Fig. 4b;
   Fig. 4d eine Fig. 4c entsprechend Detaildarstellung mit einer alternativen Ausgestaltung der Zarge.
   Fig. 5 einen Teilgrundriss des Bioreaktors im Bereich der durch die Klappe verschließbaren Stirnseite;
   Fig. 6 eine perspektivische Ansicht eines erfindungsgemäßen Bioreaktors mit geöffneter Klappe und eingesetztem Rückhaltesystem; und
   Fig. 7 ein Detail aus Fig. 6.

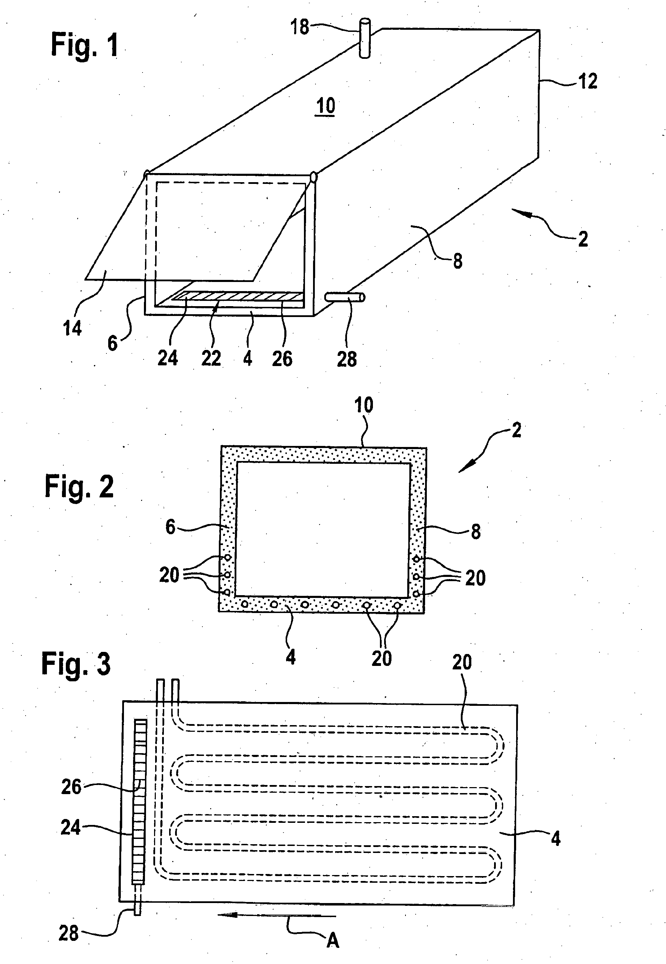

Ein Bioreaktor bzw. Biogasreaktor nach den Figuren 1 bis 3 umfasst einen quaderförmigen Faulbehälter 2, der nach Art einer Fertiggarage aus Stahlbeton besteht und sechs plane Wandelemente umfasst, nämlich eine Bodenplatte 4, zwei Seitenwände 6 und 8, eine Deckplatte 10, eine Rückwand 12 und eine offene Vorderseite, die durch eine gasdichte Klappe 14 verschließbar ist.

Die Klappe 14 ist mittels einer Hydraulik 16 betätigbar. Bei offener Klappe 14 lässt sich der Faulbehälter 2 auf einfache Weise befüllen bzw. die Restbiomasse daraus entfernen..Über einen Biogasentnahmeanschluss 18 wird das in dem Faulbehälter 2 erzeugte Biogas abgeführt. In der Bodenplatte 4 des Faulbehälters 2 und,teilweise auch in den Seitenwänden 6 und 8 kann eine Heizeinrichtung 20 nach Art einer Fußbodenheizung vorgesehen sein, mittels der die in dem Faulbehälter 2 befindliche Biomasse entsprechend temperiert werden kann. Ebenfalls in der Bodenplatte 4 integriert ist eine Sickersaft-Drainageeeinrichtung 22, die eine quer verlaufende in die Bodenplatte 4 eingelassene Rinne 24 umfasst, die durch ein Loch- oder Schlitzblech 26 abgedeckt ist. Über eine Sickersaftableitung 28 wird der sich in der Rinne 24 sammelnde Sickersaft abgeleitet. Die Bodenplatte 4 weist in Richtung des Pfeils A ein Gefälle hin zur Rinne 24 auf, so dass sich der Sickersaft in der Rinne 24 sammeln kann.

In Fig. 3 ist lediglich eine Rinne 24 dargestellt. Alternativ können mehrere solcher Rinnen vorgesehen werden, die ebenfalls quer oder in Längsrichtung angeordnet sein können.

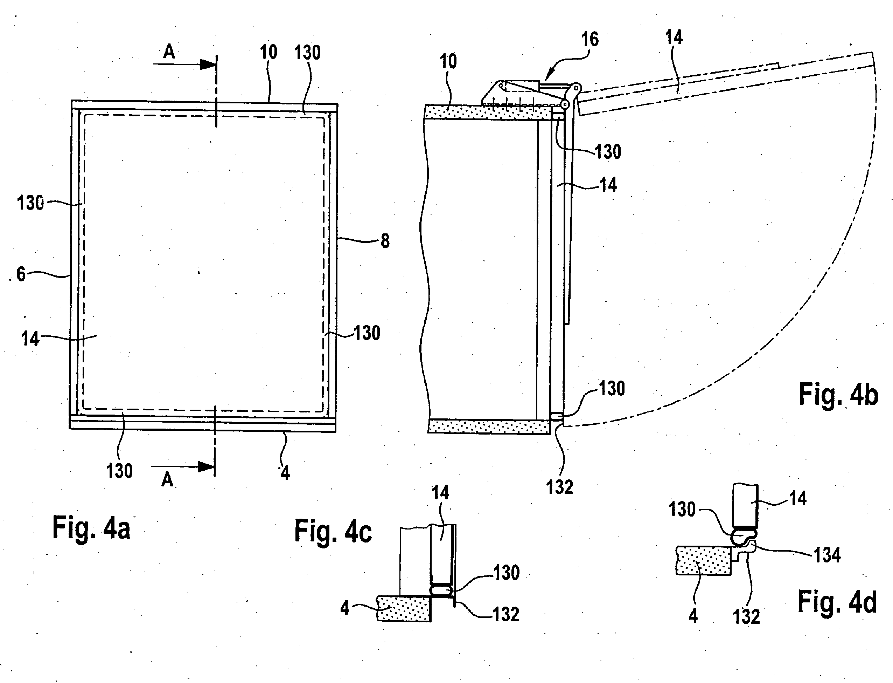

Fig. 4a zeigt eine Aufsicht auf den Faulbehälter 2 mit geschlossener Klappe 14. Fig. 4b zeigt eine Schnittansicht des Faulbehälters entlang der Ebene A-A in Fig. 4a, wobei strichliert die geöffnete Klappe 14 zusätzlich eingezeichnet ist. Im Randbereich Der Klappe 14 umlaufend ist ein Dichtungsschlauch 130 befestigt. Die Klappe 14 greift in geschlossenem Zustand in eine Zarge 132 ein - siehe Fig. 4c -, gegenüber der die Klappe 14 durch Aufpumpen des Dichtungsschlauches 130 auf 6 bar abgedichtet wird.

Fig. 4d zeigt eine alternative Ausgestaltung der Zarge 132, die einen umlaufenden Vorsprung 134 aufweist. Durch den Vorsprung 134 hintergreift der aufgeblasene Dichtungsschlauch 130 die Zarge 132, wodurch die Dichtwirkung noch erhöht wird.

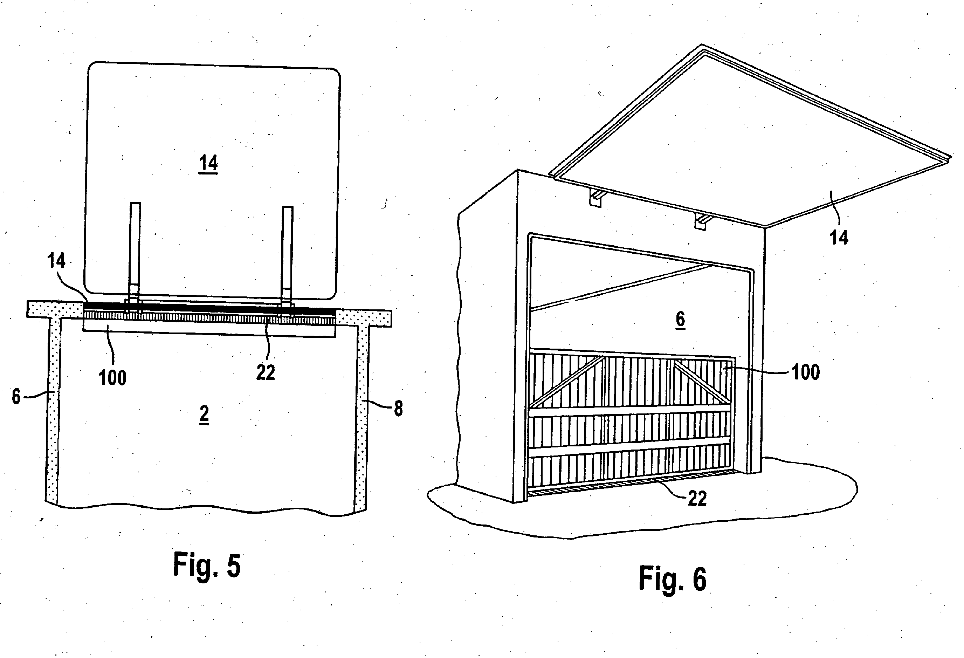

Fig. 5 zeigt einen Grundriss des vorderen Teils des Bioreaktors mit den beiden Seitenwänden 6, 8, der Sickersaft-Drainageeeinrichtung 22 und der Klappe 14, einmal im geschlossenen Zustand (in Fig. 5 dunkel dargestellt), und einmal im hochgeklappten, geöffneten Zustand (in Fig. 5 hell dargestellt). In Befüllrichtung hinter der Klappe 14 und der Sickersaft-Drainageeeinrichtung 22 wird die Rückhaltevorrichtung 100 durch vertikale Führungsschienen befestigt, indem man die plattenförmige Rückhaltevorrichtung 100, die in einer vorteilhaften Ausführung aus Holz hergestellt ist, von oben in die Führungsschienen einführt, nachdem der Bioreaktor teilweise beladen ist. Durch den zwischen der Rückhaltevorrichtung und dem Dach des Faulbehälters verbleibenden Zwischenraum kann der Bioreaktor anschließend vollständig befüllt werden.

In einer nicht gezeigten alternativen Ausführung kann die Rückhaltevorrichtung auch mechanisch, beispielsweise hydraulisch, mittels Scharnieren in der Horizontalen geschwenkt oder mittels Führungsschienen in der Vertikalen herauf- und heruntergefahren werden.

Wie in Fig. 5 zu sehen, muss sich die Rückhaltevorrichtung 100 nicht über die gesamte horizontale Breite des Faulbehälters, d. h. von der einen Seitenwand 6 zur gegenüberliegenden Seitenwand 8 erstrecken. Die durch die Klappe 14 verschließbare Beladeöffnung kann auch kleiner dimensioniert sein, so dass auch die Rückhaltevorrichtung in der Breite entsprechend schmäler ausgebildet sein kann.

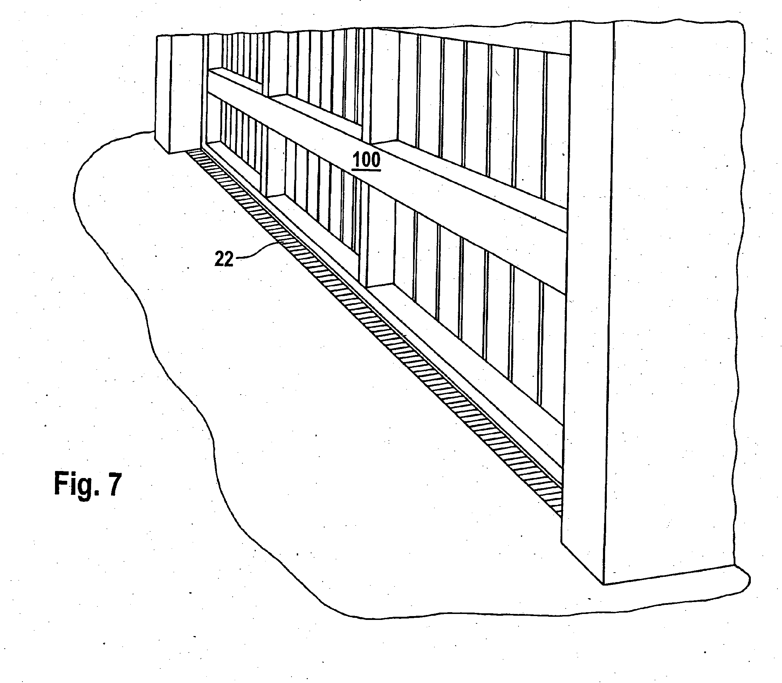

In einer in Fign. 6, 7 dargestellten weiteren Ausführungsform erstreckt sich hingegen die Rückhaltevorrichtung 100 über die gesamte Breite des Faulbehälters.

Zum Beladen wird die Klappe 14 nach oben geklappt oder hydraulisch vertikal oder horizontal verschoben und die Rückhaltevorrichtung entfernt, indem man sie in der hier gezeigten Ausführung manuell, beispielsweise mittels eines Radladers, in vertikaler Richtung aus den Führungsschienen hebt. Anschließend wird der Faulbehälter, beispielsweise mittels Radlader, zum Teil befüllt und anschließend die Rückhaltevorrichtung in umgekehrter Reihenfolge wieder eingesetzt. Durch den oben zwischen Rückhaltevorrichtung 100 und Dach des Faulbehälters verbleibenden Zwischenraum kann der Faulbehälter anschießend weiter befüllt werden. Schließlich wird die Klappe 14 geschlossen.

Durch das Eigengewicht der Biomasse wird aus dieser Sickersaft aus- und durch die in der Rückhaltevorrichtung 100 ausgebildeten Zwischenräume gepresst Vorteilhaft kann der Bioreaktor stärker mit Biomasse beladen werden als herkömmliche Reaktoren, da die Rückhaltevorrichtung das Eigengewicht abstützt. Der so ausgepresste Sickersaft sammelt sich in der Drainageeinrichtung 22, die zu diesem Zweck von der Biomasse aus gesehen hinter der Rückhalte-vorrichtung 100 angeordnet ist.

## Patentansprüche

1. Bioreaktor zur Methanisierung von Biomasse, mit
- einem Faulbehälter (2) zur Aufnahme der Biomasse, der mit einem Radlader befahrbar ist,
- einer Klappe (14), die den Faulbehälter (2) gasdicht verschließt, und
- einer Sickersaft-Drainageeinrichtung (22), die in Befüllrichtung hinter der Klappe (14) im Boden des Faulbehälters (2) angeordnet ist,
**dadurch gekennzeichnet,**
- **dass** eine plattenförmige Rückhaltevorrichtung (100) in Befüllrichtung hinter der Klappe (14) derart in dem Faulbehälter (2) angeordnet und durch vertikale Führungsschienen befestigt ist, dass die eingefüllte Biomasse sich teilweise an der Rückhaltevorrichtung (100) abstützt, und
- **dass** zwischen der Klappe (14) und der Rückhaltevorrichtung(100) die Sickersaft- Drainageeinrichtung (22) im Boden oder im Boden und den Wanden des Faulbehälters (2) angeordnet ist.

2. Bioreaktor nach einem der vorigen Ansprüche, **dadurch gekennzeichnet, dass** die Rückhaltevorrichtung bezüglich des aus der Biomasse austretenden Sickersaftes wenigstens teilweise durchlässig ist.

3. Bioreaktor nach einem der vorigen Anspruche, **dadurch gekennzeichnet, dass** die Rückhaltevorrichtung sich über die gesamte Breite des Faulbehälters erstreckt.

4. Bioreaktor nach einem der Anspruche 1 oder 2, wobei die Rückhaltevorrichtung sich nur über den Teil der gesamten Breite des Faulbehälters erstreckt, der einer durch die Klappe (14) verschließbaren Beladungsöffnung entspricht.

## Claims

1. A bioreactor for the methanation of biomass, having
- a digester (2) which is intended for accommodating the biomass and which is accessible by a wheeled loader,
- a shutter (14) which closes the digester (2) in a gas-tight manner, and
- a percolate drainage system (22) which is arranged, as seen in the filling direction, behind the shutter (14) in the floor of the digester (2),
**characterized in that**
- a panel-like retaining arrangement (100) is arranged, as seen in the filling direction, behind the shutter (14) in the digester (2) and supported therein by means of guide rails such that biomass introduced is supported in part by the retaining arrangement (100), and
- **in that** a percolate drainage system (22) is arranged in the floor or in the floor and the walls of the digester (2) between the shutter (14) and the retaining arrangement (100).

2. The bioreactor as claimed in one of the preceding claims, **characterized in that** the retaining arrangement is at least partially permeable in relation to percolate passing out of the biomass.

3. The bioreactor as claimed in one of the preceding claims, **characterized in that** the retaining arrangement extends over the entire width of the digester.

4. The bioreactor as claimed in claim 1 or 2, **characterized in that** the retaining arrangement extends only over part of the entire width of the digester, corresponding to a loading opening which can be closed by the shutter (14).

## Revendications

1. Bioréacteur pour la méthanisation de biomasse, comprenant :
- un digesteur (2) destiné à recevoir la biomasse, qui est accessible à une chargeuse sur roues,
- un volet (14) qui obture le digesteur (2) de façon étanche aux gaz , et
- un dispositif de drainage du jus de fermentation (22), qui est agencé dans le fond du digesteur (2), en arrière du volet (14) dans le sens du remplissage,
**caractérisé**
- **en ce qu'**un dispositif de retenue (100) en forme de plaque est agencé dans le digesteur (2), en arrière du volet (14) dans le sens du remplissage, et est fixé par des rails de guidage verticaux de telle sorte que la biomasse chargée s'appuie en partie contre le dispositif de retenue (100) et
- **en ce que** le dispositif de drainage du jus de fermentation (22) est agencé dans le fond, ou dans le fond et dans les parois, du digesteur (2), entre le volet (14) et le dispositif de retenue (100).

2. Bioréacteur selon l'une des revendications précédentes, **caractérisé en ce que** le dispositif de retenue est au moins partiellement perméable au jus de fermentation qui sort de la biomasse.

3. Bioréacteur selon l'une des revendications précédentes, **caractérisé en ce que** le dispositif de retenue s'étend sur toute la largeur du digesteur.

4. Bioréacteur selon l'une des revendications 1 ou 2, **caractérisé en ce que** le dispositif de retenue s'étend seulement sur la partie de la largeur totale du digesteur qui correspond à l'ouverture de chargement qui peut être obturée par le volet (14).
